# EUROPEAN PATENT SPECIFICATION

(11) **EP 0 521 590 B1**
(45) Date of publication and mention of the grant of the patent: **26.02.1997**
(21) Application number: 92250167.1
(22) Date of filing: 25.06.1992
(51) Int. Cl.: A61B 17/34, A61M 39/00, A61M 39/06

(54) **Sealing cap for guide tubes or reducing sleeves for the introduction of surgical instruments**
Verschlusseinheit für Führungsrohre oder Reduziereinsätze zur Einführung chirurgischer Instrumente
Capsule de bouchage pour tubes conducteurs ou manchon de réduction pour l'introduction des instruments chirurgicaux

(30) Priority: 02.07.1991 DE 4121829
(43) Date of publication of application: 07.01.1993
(73) Proprietor: ETHICON INC., Somerville, New Jersey 08876-0151 (US)
(72) Inventor: Brunken, Dieter, W-2359 Hüttblek (DE)
(74) Representative: UEXKÜLL & STOLBERG

(56) References cited:
- EP-A- 0 424 002
- US-A- 4 177 814
- US-A- 4 723 550
- US-A- 4 726 374
- US-A- 4 809 679
- US-A- 5 104 379

## Description

In modern laparoscopic surgery after blowing an inert gas into the abdominal cavity through a trocar, the necessary surgical instruments are introduced through a guide tube for performing minimum invasive surgery and controlled by means of an endoscope also introduced into the abdominal cavity and manipulated for carrying out the respective operation, while observing radiologically. Such laparoscopic operations are e.g. carried out in the gall bladder, stomach and intestinal tract in gynecological laparoscopy or pelviscopy, e.g. for loosening adhesions or in tubectomies.

The guide tubes hitherto used for this prupose are made from a readily sterilizable, high-quality material or chrome nickel steel alloys and have at their upper end a plastic sealing plug with a bore for the introduction of the corresponding surgical instrument.

In place of the guide tubes, it is also possible to use reducing sleeves, which have uniform external dimensions and whose internal dimensions are variable to adapt to the surgical instruments to be introduced. By a corresponding sealing cap with a central insertion opening, it is possible to insert surgical instruments with different internal diameters without inert gas pressure loss.

The known plastic sealing plugs for the aformentioned guide tubes or the proposed sealing or packing caps for the reducing sleeves suffer from the disadvantage that they can only be fitted or removed with respect to the headpiece with a certain effort, because they are sealingly applied or snapped on in the slightly expanded state. This difficult manipulatability of the sealing caps is made more difficult when using surgical instruments in that the surgeons and the operating theatre staff are forced to work with rubber gloves and the problem is exacerbated by the tissue fluid present on the gloves, so that handling is problematical when snapping on or removing the sealing caps.

EP-A-0424002 discloses a rectangular adapter seal for use with a cannula assembly. The adapter seal gives a surgeon increased flexibility in performing endoscopic procedures allowing the use of variously sized instruments through a single cannula. An end wall, which is provided with a circular opening for inserting surgical instruments, is surrounded by side walls. An essentially rectangular gripping flap extends along one of the side walls. An all-round groove for accomodating a stabilizer plate runs on the inside of the side walls, directly adjacent to the end wall. This adapter seal is used with cannula assemblies having a rectangular top side which is surrounded by outwardly extending, short side walls with smooth inner faces. For mounting the adapter seal, it has to be inserted into the space surrounded by these side walls, with the smooth outer faces of the side walls of the adapter seal engaging with the inner faces of the side walls surrounding the top side of the head piece of the cannula assembly. Thus, the known adapter seal is restricted to use with a certain type of cannula assembly only.

The problem of the invention is to propose a sealing cap for guide tubes or reducing sleeves which have a tubular head piece provided with an all-round circular flange, which sealing cap can be easily gripped and can be easily engaged on or disengaged from the all-round circular flange of the head piece of the guide tube or reducing sleeve.

According to the invention, this problem is solved by a sealing cap having the features of Claim 1. Special embodiments are given in the dependent claims.

The sealing cap according to the invention is described in greater detail hereinafter relative to the drawings, wherein show:

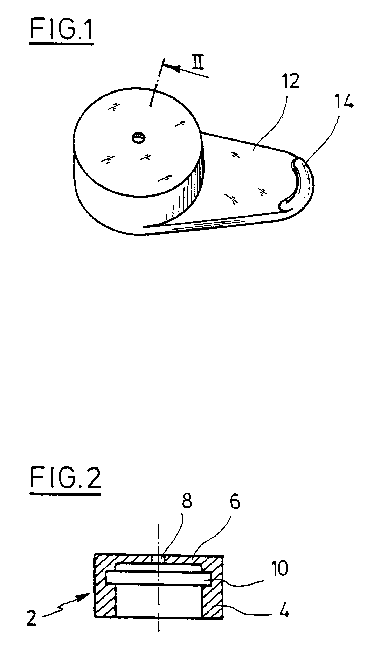

- Fig. 1:: A perspective view of the sealing cap.
- Fig. 2:: A longitudinal section through the sealing cap shown in Fig. 1.

The sealing cap 2 shown in the drawings comprises a tubular skirt 4 with a shaped on end wall 6, in which there is generally in the centre a circular insertion opening 8 for the surgical instrument. The external diameter of the skirt is e.g. 20 mm, whilst the internal diameter in the present embodiment is approximately 13.8 mm. Accompanied by slight expansion of the skirt such a cap can be applied to the headpiece of a guide tube or a reducing sleeve with an external diameter of approximately 14 mm. The cap end wall 6 shaped on in the upper area is made thinner in order to improve the guidance of the surgical instrument and e.g. in the present case has a wall thickness of 0.8 mm, whilst the skirt 4 has a wall thickness of 6.2 mm.

In the upper area of the skirt 4 is provided an all-round groove 10, which permits the snapping engagement of the cap on an all-round circular flange of the tubular headpiece of the guide tube or the reducing sleeve. In the present embodiment the internal diameter of the circular groove is approximately 16 mm and with respect to the inner face of the skirt has a depth of approximately 2.2 mm.

In a particularly preferred embodiment the circular groove 10 is at a distance from the underside of the end wall 6, which at least corresponds to the thickness of the latter. This spacing of e.g. 1 mm or more permits an easier fitting or removal by elastic deformation of the skirt, also in the upper region. If no such spacing is provided, e.g. if the groove is located immediately below the lower face of the end wall, it is less easy to remove or to lever out from the circular flange under elastic deformation the upper skirt part.

In the lower region of the skirt 4 is shaped a gripping tongue 12, whose two outer boundary edges pass approximately tangentially from two virtually diametrically facing areas of the skirt 4 and pass in rounded manner into one another on the outer gripping tongue end. In the extreme case the boundary edges can pass from the two diametrically facing areas of the skirt, but then strictly speaking would be parallel to one another in the case of a tangential direction and can then issue with a gentle bevel into the rounded portion on the outer gripping tongue end. However, it is sufficient if the boundary edges of the gripping tongue are attached roughly below the cross-section line and run tangentially in such a way that they are at an angle of approximately 15 to 20° to the cross-section line over a length of approximately 1 to 2 cm and then pass into the rounded, outer gripping tongue end. The gripping tongue length measured from the skirt 4 is preferrably 1 to 2 cm or more.

In the outer, rounded region of the outer gripping tongue end an arcuate bead 14 is shaped onto its top and permits a reliable grasping of the gripping tongue. This bead can also be shaped onto the underside and optionally additionally thereon.

Due to the fact that the boundary edges of the gripping tongue engage roughly in the vicinity of the cross-section line of the sleeve, in the case of expanding raising of the skirt the sealing cap is drawn down roughly over half the circumference of the all-round circular flange of the headpiece. The resilient elasticity in the upper region of the skirt is improved in that the circular groove 10 is located at a distance from the underside of the end wall of the cap.

The sealing cap can be made from any elastic, flexible natural or synthetic rubber-like material, preference being given to silicone rubber, particularly one having a Shore hardness of 50 to 70.

## Claims

1. Sealing cap for guide tubes or reducing sleeves for inserting surgical instruments, comprising an all-round skirt (4) extending down from the end wall of the sealing cap (2), in which skirt (4) below the end wall (6) is provided a groove (10), in the centre of the end wall (6) being provided a circular insertion opening (8) for the surgical instrument to be inserted in the guide tube or reducing sleeve, the sealing cap being made from a flexible elastomeric material, wherein on the lower edge of the skirt (4) is shaped a gripping tongue (12) whose outer boundary edges pass tangentially from two virtually diametrically facing areas of the skirt (4) and pass into one another in a rounded portion on the outer gripping tongue end,
characterized
in that the skirt (4) has an internal diameter adapted for mounting the sealing cap onto the tubular headpiece of a suitable guide tube or reducing sleeve,
in that the groove (10) is circular and is adapted to receive an all-round circular flange on the headpiece of the guide tube or reducing sleeve,
in that the circular groove (10) located below the end wall (6) of the sealing cap (2) is at a distance from the underside of the end wall (6) corresponding to at least the thickness of the latter,
in that on the gripping tongue top and/or bottom an arcuate bead (14) is shaped in the rounded region of the outer gripping tongue end, and
in that the sealing cap is made from natural or synthetic rubber-like material.

2. Sealing cap according to Claim 1 characterized in that the length of the gripping tongue (12) measured in the radial direction is approximately 1 to 2 cm.

3. Sealing cap according to Claim 1 or 2, characterized in that it is made from a silicone rubber with a Shore hardness of 50 to 70.

## Patentansprüche

1. Dichtungskappe für Führungsrohre oder Reduktionshülsen zur Einführung von chirurgischen Instrumenten, mit einer von der Stirnwand (6) der Dichtungskappe (2) herabreichenden umlaufenden Schürze (4), in der unterhalb der Stirnwand (6) eine Nut (10) vorgesehen ist, wobei in der Mitte der Stirnseite (6) eine kreisförmige Einführöffnung (8) für das in das Führungsrohr oder in die Reduktionshülse einzuführende chirurgische Instrument vorgesehen ist, wobei die Dichtungskappe aus einem flexiblen Elastomermaterial hergestellt ist, wobei am unteren Rand der Schürze (4) ein Grifflappen (12) angeformt ist, dessen äußere Begrenzungskanten tangential von zwei sich nahezu diametral gegenüberliegenden Bereichen der Schürze (4) ausgehen und am äußeren Grifflappenende in einer Abrundung ineinander übergehen,
dadurch gekennzeichnet,
daß die Schürze (4) einen Innendurchmesser hat, der zum Anbringen der Dichtungskappe an dem rohrförmigen Kopfstück eines geeigneten Führungsrohres oder einer geeigneten Reduktionshülse eingerichtet ist,
daß die Nut (10) ringförmig ist und dazu eingerichtet ist, einen umlaufenden Ringflansch an dem Kopfstück des Führungsrohres oder der Reduktionshülse aufzunehmen,
daß die ringförmige Nut (10) unterhalb der Stirnwand (6) der Dichtungskappe (2) mit einem Abstand zur Unterseite der Stirnwand (6) angeordnet ist, der mindestens der Dicke der Stirnwand (6) entspricht,
daß auf der Grifflappenoberseite und/oder -unterseite im abgerundeten Bereich des äußeren Grifflappenendes ein bogenförmiger Wulst (14) angeformt ist, und
daß die Dichtungskappe aus einem natürlichen oder synthetischen kautschukartigen Werkstoff besteht.

2. Dichtungskappe nach Anspruch 1, dadurch gekennzeichnet, daß die in radialer Richtung gemessene Länge des Grifflappens (12) etwa 1 bis 2 cm beträgt.

3. Dichtungskappe nach Anspruch 1 oder 2, dadurch gekennzeichnet, daß sie aus einem Silikonkautschuk mit einer Shorehärte von 50 bis 70 besteht.

## Revendications

1. Capsule de bouchage pour tubes conducteurs ou manchons de réduction pour l'introduction des instruments chirurgicaux, comprenant une collerette complète (4) s'étendant vers le bas depuis la paroi d'extrémité de la capsule de bouchage (2), dans laquelle collerette (4) au-dessous de la paroi d'extrémité (6) est prévue une gorge (10), dans le centre de la paroi d'extrémité (6) étant prévue une ouverture d'insertion circulaire (8) pour l'instrument chirurgical à insérer dans le tube conducteur ou manchon de réduction, la capsule de bouchage étant réalisée à partir d'un matériau électrométrique souple, dans laquelle, sur le bord inférieur de la collerette (4) est formée une languette de préhension (12) dont les bords limites extérieurs passent tangentiellement à deux zones de la collerette (4) se faisant face pratiquement diamétralement, et passent l'un dans l'autre dans une partie arrondie sur l'extrémité extérieure de la languette de préhension,
caractérisée
en ce que la collerette (4) a un diamètre intérieur conçu pour mettre en place la capsule de bouchage sur la pièce tubulaire principale d'un tube conducteur ou manchon de réduction adéquat,
en ce que la gorge (10) est circulaire et est conçue pour recevoir une bride circulaire complète sur la pièce principale du tube conducteur ou manchon de réduction,
en ce que la gorge circulaire (10) placée au-dessous de la paroi d'extrémité (6) de la capsule de bouchage (2) est à une distance du côté inférieur de la paroi d'extrémité (6) correspondant à au moins l'épaisseur de cette dernière
en ce que sur le dessus de la languette de préhension et/ou le dessous, une nervure arquée (14) est formée dans la région arrondie de l'extrémité extérieure de languette de préhension extérieure, et
en ce que la capsule de bouchage est réalisée à partir d'un matériau naturel ou synthétique semblable au caoutchouc.

2. Capsule de bouchage selon la revendication 1, caractérisée en ce que la longueur de la languette de préhension (12) mesurée dans la direction radiale est approximativement de 1 à 2 cm.

3. Capsule de bouchage selon la revendication 1 ou la revendication 2, caractérisée en ce qu'elle est réalisée à partir d'un caoutchouc silicone avec une dureté Shore de 50 à 70.
